# EUROPEAN PATENT SPECIFICATION

(11) **EP 1 198 210 B1**
(45) Date of publication and mention of the grant of the patent: **06.04.2005**
(21) Application number: 00950159.4
(22) Date of filing: 10.07.2000
(51) Int. Cl.: A61F 2/46, A61B 17/58

(54) **DIRECTING AND COMPRESSION INSTRUMENT**
STEUERUNGS- UND KOMPRESSIONSINSTRUMENT
INSTRUMENT D'ORIENTATION ET DE COMPRESSION

(30) Priority: 27.07.1999 SE 9902803
(43) Date of publication of application: 24.04.2002
(73) Proprietor: Tillander, Bo, 582 45 Linköping (SE); Palm, Lars, 589 35 Linköping (SE); Ivarsson, Ingemar, 582 72 Linköping (SE)
(72) Inventor: Tillander, Bo, 582 45 Linköping (SE); Palm, Lars, 589 35 Linköping (SE); Ivarsson, Ingemar, 582 72 Linköping (SE)
(74) Representative: Westman, Per Börje Ingemar
(86) International application number: PCT/SE2000/001474
(87) International publication number: WO 2001/006964

(56) References cited:
- EP-A1- 0 535 973
- US-A- 4 528 980
- US-A- 4 632 111
- US-A- 5 061 270

## Description

### Technical field

The present invention relates to a combined directing and compression instrument for implanting joint bowls during hip prosthesis surgery according to claim 1.

The object of the present invention is to obtain a combined directing and compression instrument for hip prosthesis surgery by means of which a more secure mounting of a hip prosthesis can be made at the exchange of a worn out hip joint.

The object of the present invention is, more specifically, to make it possible to direct the new joint bowl to be used and to compress joint bowl and joint bowl cement without shifting instrument/tool, as well as to make it possible to the use at models and designs of joint bowls present. Further, the use of a directing aid shall contribute to a higher degree of safetiness with regard to the determination of inclination and anteversion angles of the hip joint.

### Background of the invention

At hip joint surgery and thereby at exchange of a worn out hip joint to an artificial hip joint a joint bowl is cemented into the pelvis bone. To obtain a correct position of the new joint bowl in relation to the skeleton of the patient it is important that the joint bowl is being fixedly cemented in the right position. The technique of today means that one places the joint bowl in place using an underlying layer of binding cement, using a directing instrument, whereupon the directing instrument is changed to a compression instrument by means of which the binding cement is compressed underneath the joint bowl. Using the directing instrument right angles are set by means of the help of the eye, but the compression can not be made by means of the directing instrument during the curing of the cement. It has also turned out that the eye is insufficient when it comes to setting the angles.

When changing instrument from directing instrument to compression instrument when the surgeon has decided a final position for the joint bowl the pressure upon the joint bowl is let, whereby there is a risk that blood can penetrate in between the joint bowl and the bone bed in the pelvis in which case it can lead to a reduced fixation and thereby a risk for release of the joint bowl from the pelvis bone.

It is essential at the placing of the joint bowl that the determination of the inclination angle (lateral angle) and the anteversion angle (forward angle), i.e., the position of the joint bowl in the pelvis bone.

At the implantation of a hip joint prosthesis a joint bowl is fixedly cemented into the pelvis bone and a leg-shaped prosthesis provided with a ball part into the thigh bone. The ball being fixedly cemented to the thigh bone will then lead in the joint bowl and together they will form a ball joint. It is of greatest importance to obtain a correct orientation of the joint bowl in order to obtain a good movement extent and to prevent that the joint ball slips out of the joint bowl.

Using the directing instrument of today different forms of hinting or directing pins are used in one or two planes which does not provide a good carefulness at the directing of the joint bowl. One object of the present invention is thus to obtain a more careful determination of the position.

It is likewise of the great importance that one provides a heavy pressure of the joint bowl during the curing phase of the cement, about 10 minutes, in order to obtain a good fixation. During this curing phase even very small (micro) movements of the joint bowl are disadvantageous as they reduces the fixation as small inter-spaces between bone and cement and joint bowl can be formed.

Using conventional instruments one thus has to change from a directing instrument to a compression instrument where a sphere provides a pressure within the joint bowl without causing changes of the position in order not to risk these movements, a change as such means a risk to dislodge the position chosen and, which, with necessity, leads to releasing the compression.

One object of the present invention is thus to obtain a possibility of a continuous compression during the directing and compression phases.

A further object is moreover, to obtain an instrument which can be used in most, preferably all, products and designs of joint bowls by changing a directing washer for an actual joint bowl designed as such.

FR-A- 2 721 502 describes a directing instrument for joint bowls in accordance with the above where the directing instrument is directly adapted to the actual joint bowl by connecting these by means of guiding pins in order to facilitate the guiding of the joint bowl.

US-A-5,098,437 describes a directing instrument for joint bowls whereby another type of fixation of the joint bowl to the directing instrument is at hand. This fixation seems to cause considerable problems to simply release it during work for exchange to a compression instrument.

US-A-5,116,339 describes a further embodiment of a directing instrument for a joint bowl.

EP-A-0 327 509 describes a further embodiment of a directing instrument for joint bowls and joint brow for hip joint surgery whereby the directing instrument is provided with signal pins to facilitate the determination of the direction using the eye.

None of these directing instruments which have been shown in the prior art above can be said to solve the problem of compression and position and direction determination in an adequate way and among surgeons occupied with hip joint surgery it is of great importance to get a sound instrument.

An instrument according to the preamble of claim 1 is disclosed in EP-A-0 535 973.

### Description of the present invention

It has now surprisingly turned out possible to be able to solve this problem by means of the present invention which is characterized in that an attachment means is fixedly attached to the rod for receiving at least one rotatable directing pointer for position determination in two planes for adjustment of inclination angle and anteversion angle.

Further characteristics are evident from the accompanying claims.

By means of the present invention several of the problems known from prior art have been solved.

In particular, it is achieved that having the directing unit disposed towards the spherical part, it is possible to direct the joint bowl with greatest care into a favourable position and into a position with the directing unit disposed towards to handle end to obtain compression of the joint bowl and surrounding bone cement without risking dislodge the position or releasing the compression during the time as is needed for the bone cement to cure (and thereby permanently fix the joint bowl in its position).

In a particularly preferred embodiment of the invention one of said directing pointers is a level for the determination of the inclination angle of the joint bowl/hip joint prosthesis to be implanted in connection with the use of the present invention.

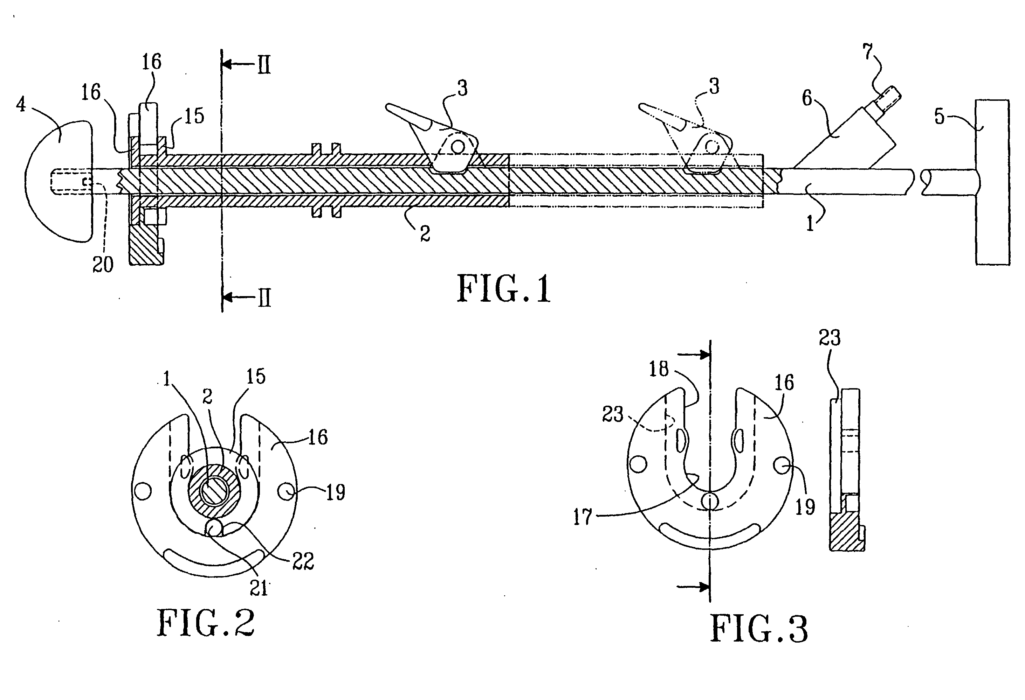

The present invention will now be described more in detail in the following with reference to the attached drawing whereby the invention is, however, not restricted to the embodiment shown but will only be restricted by the accompanying claims and within the frame work of variations made by the one skilled in the art. In the drawing
FIG. 1 shows a part of an embodiment of the invention;
FIG. 2 shows a cross-section along the line II-II of FIG. 1;
FIG. 3 shows a cross-section along the line III-III of FIG. 1 excluding the rod;

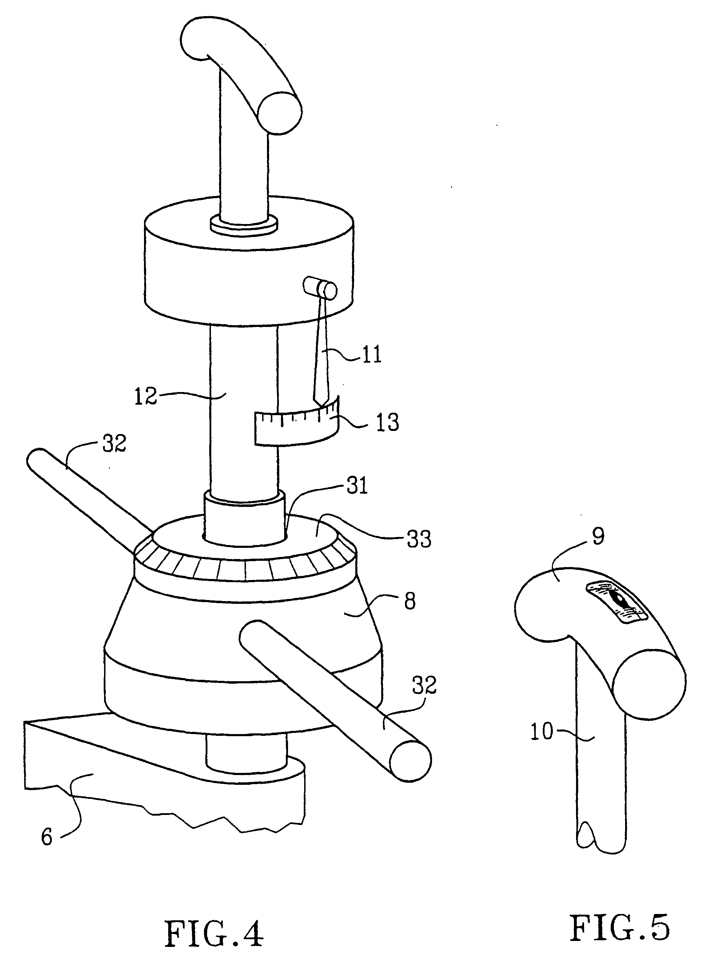

FIG. 4 shows a side perspective view of the part of the embodiment of FIG. 1 seen higher up; and
FIG. 5 shows a detail for attachment of the part shown in FIG. 4.

1 denotes a rod which suitably has a cylindrical form and is made of a material being sterilizable, preferably stainless or acid resistant steel. The rod may, however, have a quadratic cross-section. A sleeve 2 is arranged around the rod 1 whereby the sleeve 2 is arranged to be disposed along the rod 1 and to become locked to the rod by means of an eccentric pinch 3 in a desired position. The rod 1 comprises in its forward end a partly spherical part 4 which is exchangeable and adapted to the inner diameter and the shape of the joint bowl to be implanted. Joint bowls can have different depths and different extensions from case to case and for that reason the partly spherical part 4 shall be able to be exchanged. At the other, the end facing away from the partly spherical part 4 a handle 5 is detachably or fixedly arranged. Further, on the rod 1 there is an attachment means 6 fixedly arranged. The attachment means 6 comprises, in this embodiment, there is inter alia a stand 7 provided for receiving a rotatably arranged unit 8. Further, the unit 8 is provided with a centrally arranged hole 31 at its end facing away from the attachment means 6. Said hole 31 is arranged to either receive a level 9 (Fig. 7) arranged to a pin 10 being introducable into the hole 31, or a weight 11.

In an embodiment there is a weight 11 arranged to pin 12 introducable in the hole 31, whereby the weight 11 cooperates with a protractor 13. The weight 11 serves to determine the inclination angle as the eye checks contra the protractor 13 the direction/angle of the rod 1 and thereby the attachment means 6.

The rotatable unit 8 arranged on the attachment means 6 receives by an introducable joint or screw joint two directing pins 32 the direction of which determines the anteversion angle to the hip joint and for a joint bowl guided by the instrument. The angle of the directing pins to the rod 1 is determined by adjustment contra a graduated scale present on a graduated (e.g., an angle graduated washer 33 arranged above over the unit. The direction of the directing pins provide the future anteversion angle of the joint bowl.

The sleeve 2 receives in its front end two radial heels 14, 15 between which a directing washer 16 is arranged to be introduced. The directing washer 16, which is suitable made of a disposable thermoplastic material, but may also be made of a sterilizable metal for repeated use, consists of a substantially circular washer having a certain thickness into which a centrally hole 17 is provided and a groove 18n starting from said hole. The central hole 17 has such a diameter that the outer diameter of the sleeve 2 corresponds substantially therewith so that the sleeve can be placed into the hole 17 and whereby the groove 18 has such a width that this substantially corresponds to the outer diameter of the sleeve so that the sleeve can be disposed through the groove 18 to the hole 17. At the transition between groove and hole a narrowing part is arranged in order to thereby lock the washer 16 to the sleeve in the hole 17. The washer 16 has on its side facing the partly spherical part 4 two or more pins 19 arranged to be able to be received in corresponding holes 20 of the partly spherical unit 4. In a similar way there is a pin 21 arranged on the opposite side which pin is arranged to be moved into a recess 22 arranged in the heel 15 in order to lock against rotation of the washer 16. The washer 16 is at one side thereof, also provided with a groove 23 so that the outer heel 14 completely falls within the outer contour of the washer 16. This is made in order to obtain a maximal stiffness at the connection between the two units 2 and 16 in the heels 14 and 15.

At the application and directing of a joint bowl in the pelvis bone the sleeve 2 is moved forward over the rod 1 and is locked in this position whereby the pins 19 shoots into the partly spherical unit 4 and the joint bowl is directed. The fact that the joint bowl has obtained the correct direction is checked by bringing the bubble of the level 9 in the right place in the level between its markings. The level is hereby arranged to provide an inclination angle of suitable 35 to 55°, preferably 45°. Simultaneously the anteversion angle is adjusted in accordance with above by means of the directing pins 32. When this is done and the bone cement has still not been cured the compression can be maintained by means of a continued pressure in the longitudinal direction of the rod but without exposing the joint bowl and thereby the bone cement to micro movements during the curing by moving the sleeve 2 and the directing washer 16 up along the rod 1 in the direction towards the handle and thereby not providing any contact to the periphery of the joint bowl. The compression phase is exercised in this phase by the spherical part 4 only.

## Claims

1. Directing and compression instrument for implanting joint bowls during hip prosthesis surgery, comprising a central, suitably cylindrical, rod (1), a directing unit (2) being arranged and lockable to said rod as well as being disposable in a longitudinal direction along said rod, a handle (5) attached at one end of said rod (1) which handle is arranged to transmit a longitudinal force to said rod (1), a spherical part (4) attached to the rod opposite to the end of the handle (5) which spherical part is arranged to fit into a joint bowl,
**characterized in**
**that** an attachment means (6) is fixedly attached to the rod (1) for receiving at least one rotatable directing pointer (8) for position determination in two planes for adjustment of inclination angle and anteversion angle (32).

2. Directing and compression instrument according to claim 1,
**characterized in that** the directing unit comprises a lockable sleeve (2) arranged to said rod (1), as well as a directing washer (16) releasably arranged, and radially applicable to the lockable sleeve.

3. Directing and compression instrument according to claim 2,
**characterized in that** the directing washer (16) comprises at least one pin (19) arranged to be inserted into a recess (20) arranged in said spherical part (4).

4. Directing and compression instrument according to claims 2-3,
**characterized in that** the directing washer (16) is adapted to a selected joint bowl.

5. Directing and compression instrument according to claim 1,
**characterized in that** the attachment means (6) is arranged to receive a level (9).

6. Directing and compression instrument according to claim 5,
**characterized in that** the level (9) is arranged to mark using its gas bubble within range markings when the rod (1) has an angle of 35 to 55° to the horizontal plane.

## Patentansprüche

1. Steuerungs- und Kompressionsinstrument zum Implantieren von Gelenkschalen während einer Hüftprothesenoperation, mit einer zentralen, passend zylindrischen Stange, einer Steuerungseinheit (2), die an der Stange angebracht und an ihr verriegelbar ist, und die außerdem in Längsrichtung der Stange verstellbar ist, einem Handgriff (5), der an einem Ende der Stange (1) angebracht ist, und der zum Übertragen einer Längskraft auf die Stange (1) vorgesehen ist, einem sphärischen Teil (4), das an der Stange an dem vom Handgriff (5) abgewandten Ende angebracht ist, wobei das sphärische Teil dazu vorgesehen ist, in eine Gelenkschale zu passen,
**dadurch gekennzeichnet,**
**dass** an der Stange (1) eine Befestigungseinrichtung (6) fest angebracht ist, die zur Aufnahme mindestens eines drehbaren Steuerungszeigers (8) zur Positionsbestimmung in zwei Ebenen zur Einstellung des Neigungswinkels und des Winkels (32) der Bewegung im Kugelgelenk nach vorne vorgesehen ist.

2. Steuerungs- und Kompressionsinstrument nach Anspruch 1,
**dadurch gekennzeichnet,**
**dass** die Steuerungseinheit eine verriegelbare Hülse (5), die an der Stange (1) vorgesehen ist, und eine Steuerungsscheibe (16) aufweist, die an der verriegelbaren Hülse vorgesehen und radial verwendbar ist.

3. Steuerungs- und Kompressionsinstrument nach Anspruch 2,
**dadurch gekennzeichnet,**
**dass** die Steuerungsscheibe (16) mindestens einen Stift (19) aufweist, der in eine Aussparung (20) einsetzbar ist, die im sphärischen Teil (4) vorgesehen ist.

4. Steuerungs- und Kompressionsinstrument nach Anspruch 2 oder 3,
**dadurch gekennzeichnet,**
**dass** die Steuerungsscheibe (16) an eine ausgewählte Gelenkschale angepasst ist.

5. Steuerungs- und Kompressionsinstrument nach Anspruch 1.
**dadurch gekennzeichnet,**
**dass** die Befestigungseinrichtung (6) zur Aufnahme einer Wasserwaage (9) vorgesehen ist.

6. Steuerungs- und Kompressionsinstrument nach Anspruch 5,
**dadurch gekennzeichnet,**
**dass** die Wasserwaage (9) mit Hilfe ihrer Gasblase dazu dient, innerhalb der Bereichsmarkierungen anzuzeigen, wenn die Stange (1) mit der horizontalen Ebene einen Winkel von 35 bis 55° einschließt.

## Revendications

1. Instrument de direction et de compression pour implanter des logements d'articulation pendant une opération consistant à poser une prothèse de la hanche, comprenant une tige centrale (1) cylindrique de manière appropriée, une unité de direction (2) étant agencée et verrouillable sur ladite tige et pouvant être disposée dans une direction longitudinale le long de ladite tige, une poignée (5) fixée à une extrémité de ladite tige (1), laquelle poignée est agencée pour transmettre une force longitudinale à ladite tige (1), une pièce sphérique (4) fixée à la tige à l'opposé de l'extrémité de la poignée (5), laquelle pièce sphérique est agencée pour rentrer dans un logement d'articulation,
**caractérisé en ce qu'**un moyen de fixation (6) est relié fixement à la tige (1) pour recevoir au moins un pointeur de direction orientable (8) pour assurer une détermination de position en deux plans en vue d'un réglage de l'angle d'inclinaison et de l'angle d'antéversion (32).

2. Instrument de direction et de compression selon la revendication 1,
**caractérisé en ce que** l'unité de direction comprend un manchon verrouillable (2) agencé sur ladite tige (1), ainsi qu'une rondelle de direction (16) disposée amovible et applicable radialement sur le manchon verrouillable.

3. Instrument de direction et de compression selon la revendication 2,
**caractérisé en ce que** la rondelle de direction (16) comprend au moins une clavette (19) agencée pour être insérée dans un creux (20) agencé dans ladite pièce sphérique (4).

4. Instrument de direction et de compression selon les revendications 2 et 3,
**caractérisé en ce que** la rondelle de direction (16) est adaptée à un logement d'articulation sélectionné.

5. Instrument de direction et de compression selon la revendication 1,
**caractérisé en ce que** le moyen de fixation (6) est agencé pour recevoir un niveau (9).

6. Instrument de direction et de compression selon la revendication 5,
**caractérisé en ce que** le niveau (9) est agencé pour effectuer un marquage au moyen de sa bulle de gaz à l'intérieur de marquages de distance lorsque la tige (1) présente un angle de 35 à 55° par rapport au plan horizontal.
